Europäisches Patentamt

European Patent Office

Office européen des brevets

(11) Publication number: **0 123 746**
**B1**

(12) **EUROPEAN PATENT SPECIFICATION**

(45) Date of publication of patent specification: **14.10.87**

(21) Application number: **83304837.4**

(22) Date of filing: **22.08.83**

(51) Int. Cl.⁴: **A 61 L 9/03** // G11B3/00, G11B3/70

(54) Disc-playing aroma generator.

(30) Priority: **21.03.83 US 477353**

(43) Date of publication of application:
**07.11.84 Bulletin 84/45**

(45) Publication of the grant of the patent:
**14.10.87 Bulletin 87/42**

(84) Designated Contracting States:
**AT BE CH DE FR GB IT LI LU NL SE**

(56) References cited:
**FR-A-2 432 837**
**US-A-2 931 880**

(73) Proprietor: **Spector, Donald**
**380 Mountain Road**
**Union City New Jersey 07087 (US)**

(72) Inventor: **Spector, Donald**
**380 Mountain Road**
**Union City New Jersey 07087 (US)**

(74) Representative: **Cook, Anthony John et al**
**D. YOUNG & CO. 10, Staple Inn**
**London, WC1V 7RD (GB)**

Courier Press, Leamington Spa, England.

**Description**

This invention relates generally to aroma generators, and more particularly to a disc-playing aroma generator whose appearance and function are analogous to those of a phono record player, each disc to be played having a supply of liquid fragrance, whereby when an aromatic disc is inserted in the player and the player is activated, an aromatic vapor is then discharged into the atmosphere.

As used herein the term "aroma" is not limited to pleasant or savory smells, but encompasses scents that function as insecticides, air fresheners, deodorants or any other odor that acts to condition, modify or otherwise charge the atmosphere. Hence, in a disc-playing aroma generating unit, in accordance with the invention, one has a choice of discs to play, the selection depending on the atmospheric effect to be created.

The aroma of perfumes and perfume-based products such as colognes and toilet waters was originally derived from the essential oils of plants. However, since the early 19th century, chemists have succeeded in analyzing many essential oils and in creating thousands of synthetics, some simulating natural products and others yielding altogether new scents. Perfumes today are largely blends of natural and synthetic scents and of fixatives which equalize vaporization and enhance pungency. In most liquid scents the ingredients are combined with alcohol.

Olfactory organs are chemi-receptors which are stimulated by minute quantities of gases or vapors in air as low as one part in one million of air. The olfactory cells are connected with the brain by the fibers of the olfactory nerves. The perception of smell by an individual's brain is such, that if a given smell persists, the individual ceases to be aware of the smell, for he makes an accommodation to the odor which is then treated as the prevailing environment. Thus one who first enters a hospital environment becomes immediately conscious of an antiseptic odor, but his sensitivity thereto diminishes and virtually disappears if the individual remains in this environment. When, however, he leaves the hospital and is exposed to the outside atmosphere, he quickly senses this change.

Thus the operawtion of the olfactory system is such that it is highly responsive to a change in the nature or level of an aroma but is desensitized when the prevailing odor attains a steady state condition. Hence in a room having an aroma generator of the type disclosed in U.S. Patent 4,346,059 in which an aromatic vapor is continuously exuded, persons in the room subjected to the vapor cease in time to become aware of the aroma, and the generator, even though it continues to operate, serves no useful purpose.

French Patent Specification FR—A—2 432 837 discloses a form of electronic vaporiser for vaporising insecticides, air purifiers etc. in which a cartridge of the active material is positioned over a heating element.

This invention provides a disc-playing aroma generator whose appearance and function are analogous to those of a conventional phono record player, each aromatic disc to be played housing a supply of a particular liquid fragrance whereby when the disc is inserted in the player, an aromatic vapor is then discharged into the atmosphere.

In a conventional phono disc record player, the user chooses from his library of records, a recording he wishes to hear and then inserts the selected disc in a player which reproduces the record, thereby filling the room with sound. A significant feature of the present invention is that in order to fill the room with a pleasing or mood-modifying aroma, the user selects from his library of aromatic discs a disc having the desired aroma which he then inserts in the player.

Thus, as between an aromatic disc player and a conventional phono disc record player, the distinction lies in the nature of the sensory experience — the former creating an olfactory and the latter an auditory sensation. However, to carry the analogy still further, with a disc playing aroma generator in accordance with the invention, one may acquire jacketed aromatic discs in the same manner as one purchases phono disc records, the purchaser selecting discs whose aromas represent his personal preference.

More particularly, this invention provides an aromatic disc player which functions to freshen or scent the room or environment in which the unit is placed, the unit acting to periodically discharge into this environment bursts of aromatic vapor, the non-aromatic intervals therebetween having a duration sufficient to avoid desensitizing the olfactory response of the individuals exposed to the vapor.

Also the invention provides an aromatic disc and sleeve assembly which has the appearance of a conventional phono disc record.

Also the invention provides a unit of the above type which operates efficiently and reliably and which may be manufactured at low cost.

Briefly stated, these advantages are attained in a disc-playing aroma generator whose appearance and function are analogous to those of a phono disc record player, the user when playing a selected aromatic disc enjoying an olfactory rather than an auditory experience. Each aromatic disc houses a supply of a liquid fragrance whereby when the disc is inserted in the player, an aromatic vapor is then discharged into the atmosphere. The disc is formed by a circular sheet of absorbent material impregnated with the liquid fragrance and sandwiched between a pair of annular plastic films which are peripherally joined to create a central zone exposing the impregnated sheet. In the player, heated air under positive pressure is forced through the central zone to volatilize the liquid to produce a vapor which is discharged through vents in the casing of the player. The aromatic disc is packaged in a transparent sleeve having circular spindle labels on both sides thereof which overlie the central zone to impart thereto the appearance of a conventional phono record and serving to identify the fragrance.

Reference is made to the following detailed description to be read in conjunction with the accompanying drawings, wherein:

Fig. 1 illustrates, in perspective, a disc-playing aroma generator in accordance with the invention constituted by a player and two aromatic disc and sleeve assemblies and their associated outer jackets;

Fig. 2 is a section taken in a horizontal plane extending through the player to show the relationship of the disc inserted therein to the heater contained in the assembly;

Fig. 3 is a section taken in a vertical plane extending through the player;

Fig. 4 is a plane view of an aromatic disc;

Fig. 5 is a section taken in the plane indicated by line 5—5 in Fig. 4;

Fig. 6 separately shows the sleeve for the disc;

Fig. 7 is the schematic circuit diagram of the player;

Fig. 8 illustrates, in perspective, an aromatic disc player intended for a child;

Fig. 9 illustrates, in perspective an aromatic disc player intended for the interior of an automobile;

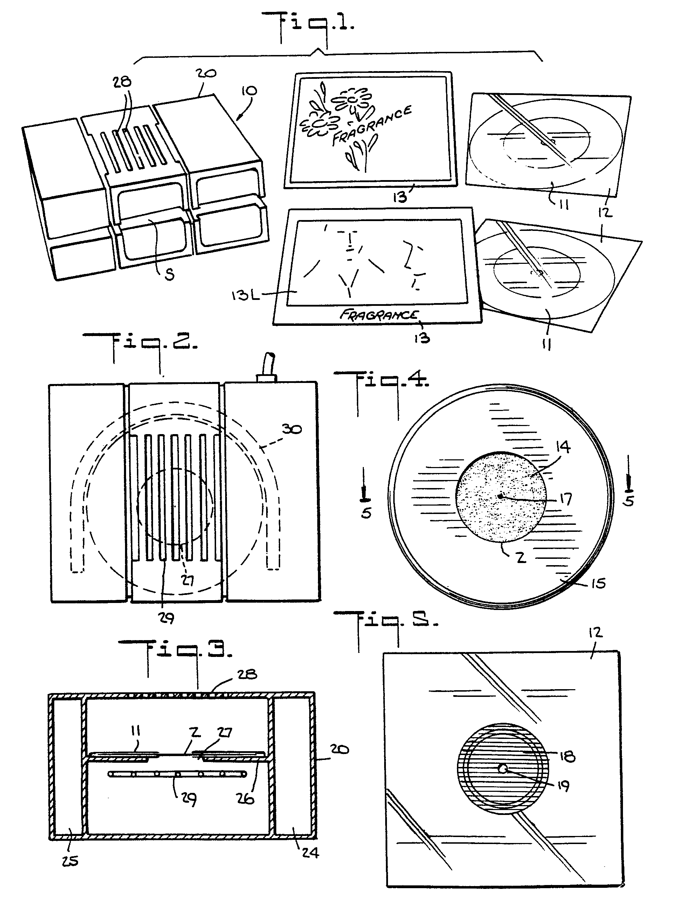

Referring now to Fig. 1, there is shown a disc-playing aroma generator in accordance with the invention consisting of a player 10 whose appearance resembles that of a conventional phono disc record player, and two aromatic discs 11 therefor. Each disc is packaged in a transparent sleeve 12 to provide a sealed assembly which is normally stored in a jacket 13. This jacket is of rigid cardboard or other suitable material comparable to that used for conventional records.

The face of the jacket has a large rectangular label 13L thereon containing a photograph or other graphic material appropriate to the identified disc fragrance. Thus if the fragrance is that of a particular flower, that flower may be photographed. If on the other hand, the fragrance is intended to create a romantic mood and has no relationship to an existing flower or other odoriforous object, the photograph may be suggestive of romance. Thus the jacket gives a potential purchaser an impression of the nature of the disc aroma and its environmental characteristics.

While only two aromatic discs are shown, in practice, a library of many discs having different aromas may be provided so that the user can select from a broad spectrum of fragrances.

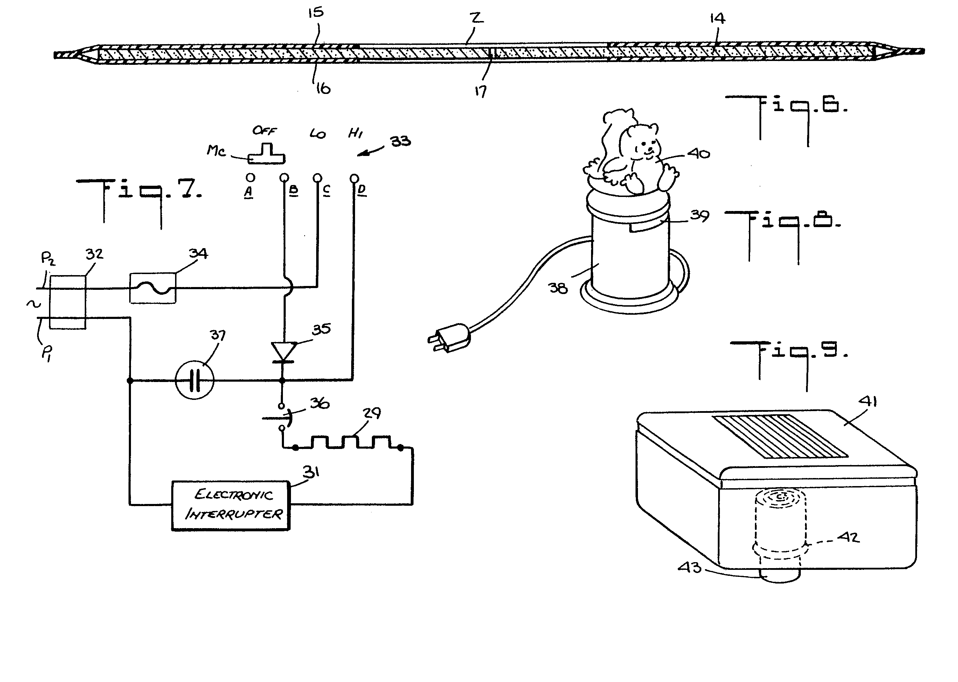

Each disc 11 as shown in Figs. 4 and 5 is composed of a circular sheet 14 of absorbent material having good wicking properties such as blotting paper, non-woven fabric or flexible open cell foam plastic material, the sheet being sandwiched between a pair of annular films 15 and 16 of black synthetic plastic material formed of polyester such as Mylar (Registered Trade Mark) PVC or other suitable plastic which is impervious to and non-reactive with the liquid fragrance which impregnates the sheet 14.

The films 15 and 16 are joined together at their periphery so that sheet 14 is exposed only in the central circular zone Z defined by the annular film. In practice, a small spindle hole 17 may be bored in the center of the sheet to relieve an excessive air pressure build-up in the player. The liquid fragrance is added to the absorbent sheet after the disc is assembled through open zone Z, the liquid being dispensed throughout the entire sheet because of its wicking properties. Thus sheet 14 represents a supply of liquid fragrance.

Disc 11 is packaged in transparent plastic sleeve 12 which shown separately in Fig. 6 is provided on either side with circular labels 18 having simulated spindle holes 19. The dimensions of the sleeve relative to the disc are such that when the aromatic disc is inserted in the sleeve, the labels then overlie zone Z on the disc so that the labels appear to cover both sides of this zone. Thus the disc in the sleeve form an assembly whose aromatic disc has spindle labels, just like a record disc.

Sleeve 12, is a square envelope having an open side to permit insertion of the disc. The open side is sealed or is provided with a pressure-operated locking strip for this purpose to prevent the loss of fragrance from the disc by evaporation during prolonged storage. By using a thermoplastic film material such as PVC for the sleeve, sealing may be effected ultrasonically or by a heated pressure bar or wheel. Alternatively, the sleeve may be provided with a flap at the open side which is closed after disc insertion.

In a conventional phono disc record, the recording on each side is a spiral track running from the periphery of the disc to a central zone, this zone being covered by a label which identifies the recording. In the present arrangement, the labels on the sleeves serve to identify the fragrance contained in the disc.

Referring now to Figs. 2 and 3 which illustrate the internal structure of player 10, it will be seen that the box-like casing 20 which may be fabricated of rigid plastic material of good structural strength is divided by vertical partitions 21 and 22 into a central chamber 23 flanked by side chambers 24 and 25. On the front wall of the case as shown in Fig. 1 there is a slot S which admits an aromatic disc 11 into central chamber 23. The side chambers are occupied by the circuit components associated with the heater element in the central chamber, which components are shown in Fig. 7.

The disc 11 inserted in the slot rests on a ledge 26 having a hole 27 therein which registers with the open zone Z of the disc. An arcuate ridge 30 on the ledge is adapted to retain the inserted disc at its proper position in the chamber. The upper wall of case 20 at the central chamber 23 is provided with vents 28 which are positioned above the inserted disc. These vents also function to simulate the loud-speaker vents in a conventional record player. Disposed below ledge 26 is an electrical heating element 29, which when activated heats the surrounding air.

When heater 29 is energized, this acts to heat the air in the confined region within central chamber 23 below the inserted disc 11. Because this heated air is confined, the resultant expansion of the air produces a positive pressure in this region, causing the heated air to force its way through open zone Z by way of the liquid impregnated sheet 14, thereby volatilizing the liquid fragrance to generate an aromatic vapor which is discharged into the atmosphere through vent 28.

The heater is preferably periodically energized so that as to produce periodic bursts of vapor separated by non-aromatic intervals having a duration sufficient to permit recovery of the olfactory response of those exposed to the vapors to avoid desensitizing this response.

The operating circuit for this purpose is shown in Fig. 7, where it will be seen that one end terminal of heater 29 is connected through an electronic interrupter 31 to one prong $P_1$ of a plug 32. This plug is insertable into a standard a—c power line outlet. A selector switch 33 is provided having fixed contacts A, B, C and D and a movable bridging connector MC. The connector is shiftable from an "Off" position at which it bridges contacts A and B, to a "Lo" position at which it bridges contacts B and C, and finally to a "Hi" position at which it bridges contacts C and D.

The other prong $P_2$ of line plug 32 is connected to contact C through a temperature sensitive cut-off device 34 which is installed in the central chamber and serves to cut-off power in the event the temperature therein rises above a pre-determined safety limit. Contact B is connected through a diode 35 to the opposite end terminal of heater element 29, a thermostatic switch 36 being interposed between diode 35 and this terminal. Contact D is connected to the junction of diode 35 and thermostatic switch 36, pilot light 37 is connected between this junction and prong $P_1$, this light going on only when the heater is energized.

Thus when movable connector MC is in its "Off" position, no power is supplied to heater element 29. When it occupies its "Lo" position, power is supplied to the heater element through diode 35 which half-wave rectifies the a—c power so that the heater is then energized by half cycles of the power and therefore operates with reduced power to generate an aromatic vapor at a relatively low rate. Thus when the unit is installed in a small room, it may be desirable to operate it at the "Lo" position.

When movable contact MC is at its "Hi" position, full power is applied to heater 29 to produce an aromatic vapor at a rapid rate. In both the "Lo" and "Hi" mode, thermostatic switch 36 cuts off when the chamber temperature exceeds a predetermined level, thereby maintaining the selected rate of vapor discharge. And in both modes, interrupter 31 acts to periodically switch on the power supplied to the heater to provide the desired pulsatory pattern of vapor-on and vapor-off to avoid desensitizing the response of those subjected to the aromatic vapor. In practice, one may dispense with this interrupter.

As shown in Fig. 8, instead of having an aroma- generator player which resembles a conventional phono record player, it may take the form of a cylindrical hollow pedestal 38 having a slot 39 therein to receive an aromatic disc 11, the pedestal housing the required heater and operating circuit. Mounted on the pedestal is a hollow animal-like figure 40 having holes therein to provide the necessary vents through which the aromatic vapor is discharged.

This toy-like player is suitable for young children who can then play, as it were, their favorite odors. It can, of course, also be used as an educational toy to teach children the distinctions between the smells of different fruits and flowers. The embodiment has play value, for children may then be asked to identify the fragrance being discharged, and a competition maybe set up to give a prize to the child who makes the greatest number of correct identifications.

In the aroma generator shown in Fig. 9, the case 41 of the player is provided with a base port 42 adapted to receive a cylindrical cigarette lighter 43 of the type conventionally used in automobiles. This lighter is provided with a spiralled heating coil which is energized by the auto battery when the lighter is pushed into a dashboard power socket, the lighter being ejected automatically when the heater coil is red hot. The player is also provided with a slot 43 to receive an aromatic coil.

This aroma generator may be mounted under the dashboard in the vehicle and activated simply by first activating the lighter in its power socket and then transferring the lighter to the player as shown in Fig. 9. While this source of heat is not in pulsatory form and of relatively short duration, it is sufficient to cause a discharge of aromatic vapor through vent 44 in the case 41 of the player in an amount which suffuses the interior of the vehicle and thereby renders its environment more pleasing or stimulating. Also for this purpose, use may be made of a de-odorizer rather than a fragrant liquid.

Thus in practice, the configuration of the player need not be that of a conventional record player but may take many other ornamental or fanciful forms as long as the player is provided with a source of heat in a confined region to produce a positive air pressure, forcing the heated air through the open zone Z of the aromatic disc.

**Claims**

1. A disc-playing aroma generator comprising:
an aromatic disc (11) constituted by a circular sheet (14) of absorbent material impregnated with a liquid fragrance and sandwiched between a pair of annular plastic films (15, 16) which are peripherally joined to create a central zone (Z) exposing said sheet, whereby said disc simulates the appearance of a conventional phono disc record; and
a player (10) having a case provided with vents (28) and having a chamber therein adapted to receive said disc so that when the disc is inserted, a confined air region is created thereby, and a heater (29) disposed in said confined region to heat the air therein whereby the resultant positive pressure forces the heated air through the sheet in said zone to volatilize the liquid impregnant and to produce an aromatic vapor that is discharged into the atmosphere through said vents.

2. An aroma generator as set forth in claim 1, in which the disc is packaged in a transparent sleeve (12) having circular spindle labels (18) on both sides thereof which when the disc is fully inserted in the sleeve overlie the central zone (Z) to simulate the spindle labels on a conventional phono disc record.

3. An aroma generator as set forth in claim 1 wherein said sheet (14) within said zone (Z) is provided with a center hole (17).

4. An aroma generator as set forth in claim 1 wherein said annular films (15, 16) are formed of flexible black plastic film material.

5. An aroma generator as set forth in claim 4, wherein said films (15, 16) are formed of polyester.

6. An aroma generator as set forth in claim 2, wherein the sleeved disc is inserted within a cardboard jacket (13) simulating a phono disc record jacket.

7. An aroma generator as set forth in claim 1, wherein said heater (29) is an electrical resistance element which is energized periodically to produce bursts of aromatic vapor interrupted by non-aromatic intervals.

8. An aroma generator as set forth in claim 1, wherein said player has a case simulating the appearance of a conventional phono disc player, the front wall of the case having a slot (S) therein to receive said aromatic disc.

9. An aroma generator as set forth in claim 8, wherein said slot leads to a ledge (26) within said chamber for supporting said disc, said ledge having a hole (27) therein which when the disc is inserted is in alignment with said zone.

10. An aroma generator as set forth in claim 1, wherein said player takes the form of a hollow pedestal which defines said chamber and has a slot (39) therein to receive said disc, said pedestal having a hollow figure (40) mounted thereon provided with said vents.

11. An aroma generator as set forth in claim 1, wherein said player has a case provided with a port (42) to receive a cylindrical automobile cigarette lighter (43) which serves as said heater.

12. An aromatic disc comprising a circular sheet (14) of absorbent material impregnated with a liquid fragrance and sandwiched between a pair of annular plastic films (15, 16) which are peripherally joined to create a central zone (Z) exposing said sheet, whereby said disc simulates the appearance of a conventional phono disc record.

**Patentansprüche**

1. Scheiben verwendender Geruchserzeuger, der umfaßt:
eine riechende Scheibe (11), die aus einem runden Blatt (14) aus absorbierendem Material besteht, das mit einem flüssigen Riechstoff imprägniert und zwischen einem Paar von ringförmigen Kunststoffilmen (15, 16) angeordnet ist, die umfangseitig verbunden sind und eine zentrale Zone (Z) bilden, in der das genannte Blatt freiliegt, wobei die genannte Scheibe im Aussehen einer üblichen Schallplatte gleicht, und

eine Abgabeeinheit (10) mit einem mit Austrittsöffnungen (28) versehenen und eine Kammer für die Aufnahme der genannten Scheibe in der Weise enthaltenden Gehäuse, daß die eingesetzte Scheibe einen Bereich mit eingeschlossener Luft schafft, und mit einem in dem genannten Bereich angeordneten Erhitzer (29) für die Beheizung der eingeschlossenen Luft, wobei der entstehende Überdruck die erhitzte Luft durch das Blatt in der genannten Zone drückt, um das flüssige Imprägnierungsmittel zu verflüchtigen und einen riechenden Dampf zu erzeugen, der in die Umgebungsluft durch die genannten Austrittsöffnungen abgegeben wird.

2. Geruchserzeuger nach Anspruch 1, wobei die Scheibe in einer transparenten Hülle (12) verpackt ist, die runde Mitteletiketten (18) auf beiden Seiten aufweist, die, wenn die Scheibe in die Hülle vollständig eingesetzt ist, die zentrale Zone (Z) überdecken, um Mitteletiketten auf einer üblichen Schallplatte zu simulieren.

3. Geruchserzeuger nach Anspruch 1, wobei das genannte Blatt (14) innerhalb der genannten Zone (Z) mit einem mittigen Loch (17) versehen ist.

4. Geruchserzeuger nach Anspruch 1, wobei die ringförmigen Filme (15, 16) aus flexiblem, schwarzen Kunststoffilmmaterial gebildet sind.

5. Geruchserzeuger nach Anspruch 4, wobei die genannten Filme (15, 16) aus Polyester gebildet sind.

6. Geruchserzeuger nach Anspruch 2, wobei die umhüllte Scheibe in eine Kartonschutzhülle (13) eingesetzt ist, die eine Schallplattenschutzhülle simuliert.

7. Geruchserzeuger nach Anspruch 1, wobei der genannte Erhitzer (29) ein elektrisches Widerstandselement ist, das periodisch mit Energie versorgt wird, um Stöße von riechendem Dampf zu produzieren, die von nichtriechenden Pausen unterbrochen sind.

8. Geruchserzeuger nach Anspruch 1, wobei die genannte Abgabeeinheit ein im Aussehen einem üblichen Schallplattenspieler gleichendes Gehäuse aufweist, wobei die Vorderseite des Gehäuses ein Schlitz (5) für die Aufnahme der genannten riechenden Scheibe enthält.

9. Geruchserzeuger nach Anspruch 8, wobei der genannte Schlitz in eine Auflageleiste (26) für die genannte Scheibe innerhalb der genannten Kammer führt, wobei die genannte Auflageleiste ein Loch (27) aufweist, das, wenn die Scheibe eingesetzt ist, mit der genannten Zone fluchtet.

10. Geruchserzeuger nach Anspruch 1, wobei die genannte Abgabeeinheit die Form eines hohlen Sockels aufweist, der die genannte Kammer bildet und einen Schlitz (39) für die Aufnahme der genannten Scheibe besitzt, wobei eine hohle Figur (40), die mit den Austrittsöffnungen versehen ist, auf den genannten Sockel montiert ist.

11. Geruchserzeuger nach Anspruch 1, wobei die genannte Abgabeeinheit ein Gehäuse mit einer Öffnung (42) für die Aufnahme eines zylindrischen Fahrzeugzigarettenanzünders (43) aufweist, der als der genannte Erhitzer dient.

12. Riechende Scheibe mit einem runden Blatt (14) aus absorbierendem Material, das mit einem flüssigen Riechstoff imprägniert und zwischen einem Paar von ringförmigen Kunststoffilmen (15, 16) angeordnet ist, die umfangsseitig verbunden sind und eine vertikale Zone (Z) bilden, in der das genannte Blatt freiliegt, wobei die genannte Scheibe dem Aussehen einer üblichen Schallplatte gleicht.

**Revendications**

1. Générateur d'arômes ayant l'aspect d'un tourne-disques, comprenant:

un disque aromatique (11), constitué d'une feuille circulaire (14) en un matériau absorbant imprégné d'un parfum liquide et inséré entre deux films plastiques annulaires (15, 16), qui sont réunis à leur partie périphérique pour créer une zone centrale (Z) mettant ladite feuille à découvert, où ledit disque simule l'aspect d'un disque phonographique classique; et

un tourne-disques (10), possédant un boîtier pourvu d'ouvertures (28), et dans lequel se trouve une chambre adaptée de façon à recevoir ledit disque, de telle sorte que, lors de l'insertion du disque, il se crée de ce fait une zone d'air fermée, et un réchauffeur (29), disposé dans ladite zone fermée, destiné à chauffer l'air qui s'y trouve, de telle sorte que la surpression qui en résulte force l'air chauffé à traverser la feuille dans ladite zone, pour provoquer la volatilisation du liquide d'imprégnation et produire une vapeur aromatique, qui est envoyée dans l'atmosphère par lesdites ouvertures.

2. Générateur d'arômes selon la revendication 1, dans lequel le disque est conditionné dans une enveloppe transparente (12) possédant des étiquettes centrales circulaires (18) sur ses deux côtés et qui, quand le disque est entièrement inséré dans l'enveloppe, recouvrent la zone centrale (Z) pour simuler les étiquettes centrales que l'on a sur un disque phonographique classique.

3. Générateur d'arômes selon la revendication 1, dans lequel ladite feuille (14), dans ladite zone (Z), possède un trou central (17).

4. Générateur d'arômes selon la revendication 1, dans lequel lesdits films annulaires (15, 16) sont constitués d'un matériau pour film plastique, noir, souple.

5. Générateur d'arômes selon la revendication 4, dans lequel lesdits films (15, 16) sont en polyester.

6. Générateur d'arômes selon la revendication 2, dans lequel le disque entouré de l'enveloppe est inséré dans une pochette en carton (13), qui simule une pochette d'un disque phonographique.

7. Générateur d'arômes selon la revendication 1, dans lequel ledit réchauffeur (29) est un élément résistif électrique, qui est périodiquement excité pour produire des bouffées de vapeur aromatique, interrompues par des intervalles ne dégageant pas d'arômes.

8. Générateur d'arômes selon la revendication 1, dans lequel ledit tourne-disques possède un boîtier qui simule l'aspect d'un tourne-disques pour disques phonographiques classique, la paroi avant du boîtier possédant une fente (S) pour recevoir ledit disque arômatique.

9. Générateur d'arômes selon la revendication 8, dans lequel ladite fente conduit à un rebord (26) se trouvant dans ladite chambre et destiné à supporter ledit disque, ledit rebord possédant un trou (27) qui, lors de l'insertion du disque, est en alignement avec ladite zone.

10. Générateur d'arômes selon la revendication 1, dans lequel ledit tourne-disques prend la forme d'un socle creux qui définit ladite chambre et possède une fente (39) destinée à recevoir ledit disque, ledit socle étant surmonté d'un personnage creux (40), pourvu desdites ouvertures.

11. Générateur d'arômes selon la revendication 1, dans lequel ledit tourne-disques possède un boîtier pourvu d'un orifice (42) destiné à recevoir un allume-cigare cylindrique pour automobile (43), qui sert de réchauffeur.

12. Disque aromatique comprenant une feuille circulaire (14) en un matériau absorbant imprégné d'un liquide odorant et inséré entre deux films plastiques annulaires (15, 16) qui sont réunis à leur partie périphérique pour créer une zone centrale (Z) mettant à découvert ladite feuille, où ledit disque simule l'aspect d'un disque phonographique classique.

0 123 746

Fig.1.

Fig.2.

Fig.4.

Fig.3.

Fig.S.

1

15  2  14

16  17

OFF  Lo  Hi

Mc  33

Fig.7.

A  B  C  D

40

39

Fig.8.

38

P₂  32  34

35

37

36  29

31

ELECTRONIC
INTERRUPTER

P₁

Fig.9.

41

42

43

2

0 123 746